# DEMANDE DE BREVET EUROPEEN

(11) **EP 4 151 202 A1**
(43) Date de publication de la demande: **22.03.2023**
(21) Numéro de dépôt: 22195690.7
(22) Date de dépôt: 14.09.2022
(51) Int. Cl.: A61K 8/64, A61Q 19/00

(54) **COMPOSITION POUR PRÉVENIR ET/OU TRAITER LES VERGETURES BLANCHES**

(30) Priorité: 15.09.2021 FR 2109660
(71) Demandeur: LSI Silderma Ltd, Cork T23AT2P (IE)
(72) Inventeur: DIEHL, Christian, 5008 Córdoba (AR); CHAMI DE DIEHL, Silvia, 5008 Córdoba (AR)
(74) Mandataire: Cabinet Laurent & Charras

(57) **Abrégé**

Composition comprenant du palmitoyl tripeptide -5 comme seul et unique principe actif dermatologique, lequel représente entre 0.01% et 0.1% en masse par rapport à la masse totale de la composition, pour utilisation dans la prévention et/ou le traitement des vergetures blanches

## Description

### DOMAINE TECHNIQUE DE L'INVENTION

La présente invention concerne le domaine de la prévention et/ou du traitement des vergetures, plus spécifiquement des vergetures blanches. Plus particulièrement, l'invention concerne une composition comprenant, en tant que principe actif dermatologique, du palmitoyl tripeptide -5, pour utilisation dans la prévention et/ou le traitement des vergetures blanches.

La présente invention, concerne également un procédé cosmétique d'application de ladite composition pour prévenir et/ou traiter les vergetures blanches.

### ARRIERE-PLAN TECHNOLOGIQUE

Les vergetures sont des lésions dermiques, bénignes qui apparaissent par exemple lors d'une grossesse, à l'adolescence, ou à la suite d'une prise de poids rapide, notamment au niveau du ventre, des fesses, des cuisses ou des seins.

Cliniquement, on distingue deux formes physiologiques de vergeture : la vergeture rouge qualifiée aussi de rosée et la vergeture blanche. Ces formes physiologiques correspondent à des stades évolutifs différents de la lésion dermique. Les vergetures rouges et blanches se traduisent par des caractéristiques histologiques, biochimiques et un état physiopathologique différents.

Au stade évolutif initial, l'érythème est dans sa phase aiguë, sous forme de vergeture rouge.

La vergeture rouge est visible sous la forme de stries linéaires rouges ou violacées, voire pourpres sur la peau. En effet, la vergeture rouge se caractérise par une augmentation de la micro-vascularisation qui contribue à sa couleur érythémateuse rouge. Histologiquement, la vergeture rouge présente un œdème dermique avec une prédominance de fines fibres élastiques dermiques, amincissant le derme. Biochimiquement, la vergeture rouge se caractérise par une altération du collagène et des fibres élastiques, mettant en évidence des changements structurels.

Au cours du temps, l'érythème entre dans la phase chronique cicatricielle, sous forme de vergeture blanche.

La vergeture blanche est visible sous la forme d'un tissu atrophique, générant une marque permanente ridée, souvent linéaire, hypo-pigmentée, déprimée et flasque, qui est visuellement proche d'une cicatrice.

Biochimiquement, l'apparition des vergetures blanches résulte d'une diminution de l'expression des gènes, comme le procollagène de type I et III, l'élastine et la fibronectine, provoquée notamment par une altération du mécanisme des fibroblastes. En effet, les fibroblastes, résidents du derme, en assurent la cohérence et la souplesse, notamment au travers de la voie de signalisation du facteur de croissance TGF-β. Ce dernier est le régulateur principal de la matrice extracellulaire.

Histologiquement, au niveau de l'épiderme, les vergetures blanches se caractérisent par une atrophie avec perte des crêtes épidermiques, et une réduction des mélanocytes. Au niveau du derme, les vergetures blanches présentent du collagène et des fibres élastiques denses, tassées et alignées parallèlement à la jonction épidermique dermique, et une micro-vascularisation réduite entrainant leur couleur pâle.

Les vergetures sont des affections cutanées courantes, à l'aspect inesthétiques, qui sont à l'origine d'un mal-être chez les personnes touchées.

C'est pourquoi, il convient de trouver des solutions pour traiter les vergetures et éviter leur apparition ou développement.

Plusieurs traitements contre les vergetures existent à ce jour, sans distinctions entre les vergetures rouges et blanches, pourtant différentes histologiquement et biochimiquement. Le manque de spécificité des traitements existants, en fonction du type de vergeture, fait que ces traitements existants ne sont pas satisfaisants.

Sur le marché actuel, on distingue la technique mécanique et la technique chimique de traitement des vergetures.

Parmi les techniques mécaniques, on citera par exemple la micro- dermabrasion, la radiofréquence, le traitement par laser fractionné, laser à diode, laser excimère au chlorure de xénon, laser vasculaire, la lumière pulsée intense, l'infrarouge, la lumière UV, mais aussi la thérapie percutanée d'induction de collagène, ou du plasma riche en plaquette.

Néanmoins, ces techniques présentent plusieurs effets secondaires indésirables, dont l'hyperpigmentation post inflammatoire, l'hyperpigmentation transitoire, l'apparition d'érythème, d'œdème, d'intolérance cutanée ou d'ecchymose. En outre, ces techniques ne sont pas toutes adaptées à tout type de sujet, tel que par exemple les femmes enceintes qui sont pourtant grandement touchées par l'apparition des vergetures.

Parmi les techniques chimiques par application topique, on citera par exemple le peeling chimique, impliquant l'application d'acide trichloracétique ou d'acide glycolique. Toutefois, le peeling chimique n'est pas satisfaisant pour traiter les vergetures, en particulier les vergetures blanches. Le peeling chimique présente, en outre, des effets secondaires indésirables comme une hyperpigmentation post-inflammatoire. Parfois, pour augmenter son efficacité, le peeling chimique à l'acide glycolique est réalisé en combinaison avec de la trétinoïne, qui est déconseillée pendant la grossesse et l'allaitement. Ainsi, le peeling chimique à l'acide glycolique combiné à la trétinoine ne permet pas de traiter tous les sujets avec vergeture.

D'autres composés chimiques, d'application topique associée à un massage cutané, sont connus pour traiter les vergetures en général. On citera par exemple des crèmes contenant de l'acide hyaluronique ou des extraits de Centella *asiatica,* l'application d'huile d'amande douce ou d'huile d'olive. Toutefois, à ce jour, l'action chimique sur les vergetures n'a pas été dissociée de l'effet mécanique lié au massage répété de la peau.

Le document US20120094919A1 décrit l'utilisation de dérivés de tripeptide de formule spécifique, dont ne fait pas partie le palmitoyl tripeptide 5, en particulier pour réduire les vergetures sans faire de distinction entre vergeture blanche et vergeture rouge. Le tripeptide préféré est le tripeptide 8 utilisé dans tous les exemples. L'exemple 6 mentionne le palmitoyl tripeptide 5 parmi une longue liste d'ingrédients cosmétiquement actifs.

CN110025508A décrit une composition cosmétique pour le traitement des vergetures sans faire de distinction entre vergeture blanche et vergeture rouge. La composition comprenant l'association d'huile de graines de Lotus, d'avocat, d'huile d'argan et de palmitoyl tripeptide 5. La concentration de palmitoyl tripeptide 5, est comprise entre 1 et 5% en poids de la composition. Il est dit que ce peptide agit pour la production de collagène et a notamment un effet sur les rides.

CN113332193A décrit une composition pour le traitement des vergetures sans faire de distinction entre vergeture blanche et vergeture rouge. La composition associe différents ingrédients dont un extrait de Centella asiatica, carnosine, palmitoyl tripeptide-5, palmitoyl hexapeptide-11. Le palmitoyl tripeptide-5 est décrit pour son activité sur la production de collagène.

Le document CN110974716A décrit une composition pour le traitement des vergetures associant un dérivé de silanol combiné à des composés transdermiques tels que acetylchitosamine et hectorine. Du palmitoyl tripeptide-5 est utilisé dans un des exemples, en tant qu'excipient. Ce document fait la distinction entre vergeture blanche et vergeture rouge sans pour autant qu'il soit indiqué si la composition illustrée agisse sur l'une, l'autre ou les 2 catégories de vergetures.

XP002774477 (MINTEL) décrit une composition pour traiter les vergetures comprenant parmi une longue liste d'ingrédients, du palmitoyl tripeptide 5. Il est dit que l'association des antioxydants, des peptides et des extraits naturels de plante permet de réduire la taille et favoriser la décoloration des vergetures.

Ainsi, les compositions cosmétiques anti-vergetures, à action chimique connues, n'apportent pas pleine satisfaction pour prévenir ou traiter les vergetures blanches, notamment à cause des effets secondaires.

Il subsiste donc un besoin de mettre au point de nouvelles compositions, pour traiter et/ou prévenir la formation, et le développement des vergetures blanches, en particulier des seules vergetures blanches, qui soient utilisables facilement par tous types de sujets incluant les femmes enceintes.

### DESCRIPTION DE L'INVENTION

De manière surprenante, le Demandeur a constaté que l'utilisation d'une composition comprenant du palmitoyl tripeptide -5 permet de surmonter les inconvénients précités pour prévenir et/ou traiter spécifiquement les vergetures blanches.

A cet effet, la présente invention concerne une composition comprenant, en tant que principe actif dermatologique, du palmitoyl tripeptide -5, pour utilisation dans la prévention et/ou le traitement des vergetures blanches, en pratique des seules vergeture blanches.

On entend par le terme « principe actif dermatologique », un composé destiné à être appliqué sur la peau et qui possède des propriétés biologiques qui sous -tendent un effet physiologique. Le principe actif est à différencier des « excipients » de la composition.

On entend par le terme « excipient », tous composés qui confèrent à la composition ses propriétés physicochimiques, telles que sa consistance, sa galénique et sa capacité à délivrer ledit principe actif dermatologique en étant inerte vis-à-vis de son effet physiologique.

On entend par l'expression « prévention des vergetures blanches », le fait d'éviter l'apparition de nouvelles vergetures blanches à la surface de la peau.

On entend par l'expression « traitement des vergetures blanches », le fait de limiter leur développement, leur croissance et leur étendue à la surface de la peau.

Selon un mode de réalisation préféré de la composition de l'invention, ledit palmitoyl tripeptide -5 représente entre 0.01% et 0.1%, de préférence 0,025% en masse par rapport à la masse totale de la composition.

Le palmitoyl tripeptide -5 a pour avantage d'être facilement mis en formulation dans une composition, d'être non cytotoxique, biocompatible et accepté par la règlementation des produits cosmétiques en vigueur. De plus, l'action du palmitoyl tripeptide -5, même à des concentrations inférieures à 0,1%, montre des résultats satisfaisants, à l'encontre des vergetures blanches, comme indiqué par la suite.

Selon un mode préféré de réalisation de l'invention, la composition comprend comme seul principe actif dermatologique, ledit palmitoyl tripeptide -5.

Préférentiellement, ledit palmitoyl tripeptide -5 consiste en un tripeptide de valine- lysine-valine, il permet avantageusement de traiter et/ou prévenir les vergetures blanches.

Selon un mode de réalisation préféré, la composition utilisée pour prévenir et/ou traiter les vergetures blanches, se présente sous une forme galénique adaptée pour un usage topique. En d'autres termes, ladite composition est sous une forme galénique qui permet au principe actif dermatologique d'agir localement, en ciblant les vergetures blanches, en particulier à l'endroit d'application de la composition sur la peau.

De préférence, la composition utilisée pour prévenir et/ou traiter les vergetures blanches, se présente sous une forme galénique adaptée pour une voie d'administration cutanée. En d'autres termes, la composition est administrée à la surface de la peau. Dans ce cas, la forme galénique peut être par exemple une crème, un gel, une huile, une lotion, une pommade, une mousse.

Selon un autre mode de réalisation, la composition, utilisée pour prévenir et/ou traiter les vergetures blanches, se présente sous une forme galénique adaptée pour une voie d'administration transcutanée. En d'autres termes, la composition est appliquée à travers la peau, par injection sous cutanée, par exemple avec une aiguille contenant la composition sous forme liquide, par exemple sous forme d'une solution.

De préférence, la composition utilisée pour prévenir et/ou traiter les vergetures blanches, se présente sous la forme de crème, de gel, d'huile, de lotion, de solution, de pommade, de mousse, d'émulsion. Cette forme galénique a pour avantage de permettre une application facile et ciblée sur les vergetures blanches.

Selon un mode de réalisation particulier, la composition selon l'invention est une composition cosmétique.

En d'autres termes, l'invention concerne donc une utilisation cosmétique du palmitoyl tripeptide -5, en tant que principe actif dermatologique, pour prévenir et/ou traiter les vergetures blanches.

Selon un mode de réalisation particulier, l'invention concerne une composition, utilisée pour prévenir et/ou traiter les vergetures blanches, qui comprend en pourcentage massique par rapport à sa masse totale, égale à 100% :
- entre 70% et 80% d'eau ;
- entre 1% et 10% de glycérine ;
- entre 1% et 10% de triglycéride caprylique et/ou caprique ;
- entre 1% et 10% de néopentanoate d'isostéaryle ;
- entre 1% et 10% de sorbitane stéarate
- entre 1% et 10% de polysorbate 60 ;
- entre 1% et 10% de diméthicone ;
- entre 1% et 10% d'alcool cétylique ;
- entre 0,5% et 5% de pentylène glycol ;
- entre 0,1% et 1% de cire d'abeille;
- entre 0,1% et 1% de chlorphénésine ;
- entre 0,1% et 1% de benzoate de sodium ;
- entre 0,1% et 1% d'alkyle acrylate/ C10-30 ;
- entre 0,05% et 0,5% de cymène 5-ol ;
- entre 0.01% et 0.1% de palmitoyl-tripeptide-5 ;
- entre 0.001% et 0.01% d'hydroxyde de sodium; et
- entre 0.0005% et 0.005% d'acide citrique.

La présente invention concerne également un procédé cosmétique, pour la prévention et/ou le traitement des vergetures blanches, selon lequel on applique sur les vergetures blanches une composition selon l'invention. Dans le cas d'une application de la composition de l'invention par voie d'administration cutanée, un léger massage permet sa pénétration dans les différentes couches de la peau afin notamment que le principe actif dermatologique, en l'espèce le palmitoyl-tripeptide-5, atteigne l'épiderme, le derme, voire l'hypoderme. Dans le cas d'une application de la composition par voie transcutanée, l'injection ciblée de la composition permet de libérer directement le palmitoyl-tripeptide-5 dans l'épiderme, le derme, voire l'hypoderme.

Selon un mode de réalisation préféré du procédé cosmétique de l'invention, on applique deux fois / jours, de préférence 1 fois le matin et 1 fois le soir, sur les vergetures blanches, une fois la peau nettoyée, ladite composition. Ainsi, cette méthode d'application, permet au d'obtenir l'effet physiologique du palmitoyl-tripeptide-5 sur les vergetures blanches, à la fois au cours de la journée et pendant la période de sommeil des utilisatrices. En conséquence, cette posologie permet d'être efficace sur les 24h de la journée dans la prévention et/ou le traitement des vergetures blanches.

Selon un autre mode de réalisation du procédé cosmétique de l'invention, on applique une quantité efficace de ladite composition de l'invention sur les vergetures blanches.

On entend par le terme « quantité efficace », la quantité nécessaire de la composition, à appliquer sur les vergetures blanches, pour obtenir un effet physiologique significatif qui permet de prévenir leur apparition et/ou permet de les traiter en ralentissant leur développement.

Selon un mode de réalisation privilégié, on applique entre 0,02 et 0,04 mL de la composition de l'invention / cm² de peau concernée.

Il ressort de ce qui précède que la composition comprenant le palmitoyl-tripeptide-5, avantageusement cosmétique, permet de prévenir et/ou traiter spécifiquement les vergetures blanches.

En conséquence, la composition de l'invention permet d'éviter la formation et le développement des vergetures blanches. L'usage de la composition de l'invention permet d'éliminer l'aspect inesthétique des vergetures blanches et de réduire leur croissance et leur extension en surface sur la peau.

D'autres caractéristiques et avantages de l'invention ressortiront de la description détaillée qui va suivre des modes de réalisation non limitatifs de l'invention, en référence à la figure annexée, dans laquelle :

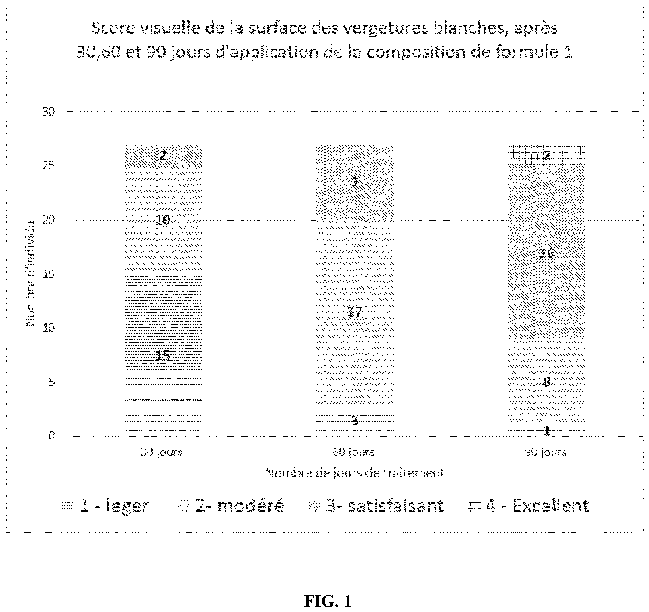

- la figure 1 représente, l'évaluation de l'amélioration de la surface dermique des vergetures blanches, observée après 30 jours, 60 jours ou 90 jours d'application par voie cutanée de la composition selon l'invention.

### EXEMPLE DE REALISATION

Dans cet exemple, la composition selon l'invention, pour son utilisation dans la prévention et/ou le traitement des vergetures blanches, est de formule 1.

La formule 1 comprend en pourcentage massique par rapport à sa masse totale, égale à 100% :
- 75,471426 % d'eau;
- 5,1% de glycérine ;
- 4% de triglycéride caprylique et/ou caprique ;
- 3,75% de néopentanoate d'isostéaryle ;
- 3% de sorbitane stéarate ;
- 2,5% de polysorbate 60 ;
- 2% de diméthicone ;
- 1,5% d'alcool cétylique ;
- 1% de pentylène glycol ;
- 0,75% de cire d'abeille ;
- 0,3% de chlorphénésine ;
- 0,3% de benzoate de sodium ;
- 0,2% d'alkyle acrylate/ C10-30 ;
- 0,1% de cymène 5-ol ;
- 0,025% de palmitoyl-tripeptide-5 ;
- 0,002574% d'hydroxyde de sodium, et
- 0,001% d'acide citrique.

Dans la formule 1 ci-dessus, tous les composés sont considérés comme des excipients de formulation, sauf le palmitoyl-tripeptide-5 qui consiste en le seul principe actif dermatologique.

### Evaluation de l'efficacité et de la tolérance de l'application d'une composition cosmétique selon la formule 1 susmentionnée pour traiter les vergetures blanches

Pour évaluer l'efficacité et la tolérance d'application de la composition de l'invention, 27 individus ont appliqué, matin et soir, pendant 90 jours, sur une zone de peau nettoyée avec vergetures blanches existantes, soit une composition selon l'invention, soit une composition « placebo ».

Dans le protocole d'étude, les individus sont constitués à la fois d'hommes et de femmes. Pour chacun d'entre eux, aucun traitement à l'encontre de leurs vergetures blanches n'a été réalisé dans les 4 semaines avant le démarrage du protocole d'étude, afin d'éviter toutes interactions ou effets liés à des traitements antérieurs.

La composition cosmétique selon l'invention est de formule 1 et comprend le palmitoyl-tripeptide-5. La composition « placebo » est de formule identique à la formule 1, mais sans le palmitoyl-tripeptide-5.

Chez chaque individu, la composition « placebo » a été appliquée sur une première moitié de la zone constituée par les vergetures blanches, et la composition de l'invention sur l'autre moitié de ladite zone. L'application cutanée sur les différentes zones de vergetures blanches a été accompagnée d'un léger massage pour favoriser la pénétration dans la peau de chacune des deux compositions. En moyenne, il a été appliqué entre 0,02 et 0,04 mL de composition / cm² de peau avec vergetures blanches.

L'évolution de la surface des lésions des vergetures blanches a été évaluée après 30 jours, 60 jours et 90 jours d'applications, pour la zone traitée avec la composition de l'invention par rapport à la zone traitée avec la composition placebo.

Le tableau 1 ci-dessous montre le pourcentage moyen d'amélioration observée à la surface de chacune des vergetures blanches évaluées, en fonction du nombre de jour de traitement et pour chacune des compositions testées.

L'analyse de la surface des vergetures blanches est une analyse planimétrique, réalisée avec le logiciel ImageJ, qui permet, au cours du temps, d'évaluer l'étendue de surface d'une vergeture blanche déterminée, par rapport à sa surface initiale de départ pré-protocole. La surface initiale de départ qu'occupe une vergeture déterminée sur la peau correspond à une surface de référence définie comme étant de 100%.

Une diminution de la surface de peau qu'occupe une vergeture blanche déterminée, au cours du temps, se traduit par un pourcentage d'amélioration de la lésion dermique qui est positif et croissant.

Au contraire, une augmentation de la surface de peau qu'occupe une vergeture blanche déterminée, au cours du temps, se traduit par un pourcentage d'amélioration de la lésion dermique qui est décroissant, faible, voire négatif.

**Tableau 1**

| | % d'amélioration de la surface de la lésion de vergetures blanches | |
|---|---|---|
| Nombre de jours de traitement | Avec la composition de l'invention de formule 1 | Avec la composition " placebo" |
| 30 jours | 16,63% | -6,07% |
| 60 jours | 36,69% | -1,12% |
| 90 jours | 45,96% | 3,98% |

Ce tableau 1 montre que l'application de la composition de formule 1 de l'invention améliore significativement la lésion de vergeture blanche considérée à la surface de la peau, alors que ceci n'est pas le cas avec la composition « placebo ».

Les résultats montrent par exemple qu'après 90 jours de traitement avec la composition de l'invention, en moyenne sur l'ensemble des vergetures blanches initiales considérées, les vergetures blanches perdent jusqu'à 45,96% de leur surface, par rapport à la surface qu'elles occupaient initialement sur la peau avant le traitement. La zone de peau sur laquelle elles s'étendaient initialement a diminuée. L'usage de la composition de l'invention entraine donc une diminution significative de l'étendue des vergetures blanches sur la peau. Ainsi, la présence du palmitoyl-tripeptide-5 joue son rôle de principe actif dermatologique dans la composition de l'invention en améliorant l'état et l'étendue de la surface qu'occupent les lésions de vergetures blanches.

Il est à noter qu'aucun effet secondaire n'a été observé, pendant toute la période d'application de la composition de formule 1 ou de la composition placebo. On entend par « effet secondaire » des brûlures, irritations, démangeaisons ou tout autre type de modification de la peau au niveau de la surface d'application de la composition de l'invention. Ainsi, le palmitoyl-tripeptide-5, en tant que principe actif dermatologique, en association avec les excipients, est bien toléré en application cutanée.

En plus de l'analyse planimétrique de la surface occupée par une vergeture blanche donnée par le logiciel, une évaluation visuelle de la lésion sur la zone traitée par la composition de l'invention a été réalisée sur chaque patiente. L'évaluation visuelle, après 30, 60 et 90 jours d'application, est déterminée en indiquant un score allant de 1 à 4 pour l'aspect visuel de la vergeture blanche. On entend par le terme « aspect visuel » d'une vergeture blanche sa longueur, sa largeur, l'intensité de la couleur blanche, sa surface totale.

Le score de 1 signifie une légère amélioration visuelle de l'aspect de la vergeture blanche, de l'ordre de 0 à 25% par rapport à la vergeture blanche initiale sans traitement.

Le score de 2 signifie une amélioration visuelle modérée, de l'ordre de 25 à 50% par rapport à l'aspect initial de la vergeture blanche sans traitement.

Le score de 3 signifie une amélioration visuelle satisfaisante, de l'ordre de 50 à 75% par rapport à l'aspect initial de la vergeture blanche sans traitement.

Le score de 4 signifie une excellente amélioration visuelle, supérieure à 75% par rapport à l'aspect initial de la vergeture blanche sans traitement.

La figure 1 récapitule les scores évalués pour chaque individu, en fonction du nombre de jours de traitement, après application, matin et soir, de la composition de l'invention de formule 1 sur une zone de peau présentant des vergetures blanches.

Les résultats montrent qu'après 30 jours d'application de la composition de l'invention, 1/3 des patients (10 individus) présentent une amélioration visuelle modérée et plus de la moitié des patients (15 individus) présentent une amélioration légère de la lésion dermique de la vergeture blanche évaluée.

De la même manière, après 60 jours d'application, quasiment 2/3 des patients (17 individus) présentent une amélioration dermique modérée de la lésion de vergeture blanche. et 1/4 des patients (7 individus) présentent une amélioration visuelle satisfaisante de la lésion dermique de vergeture blanche.

Après 90 jours d'application, les résultats montrent que plus de la moitié des personnes (16 individus) présentent une amélioration visuelle satisfaisante des vergetures et deux personnes présentent une excellente amélioration visuelle de la lésion des vergetures blanches.

En d'autres termes, les résultats de la figure 1 montrent, qu'au cours du temps, l'application de la composition de formule 1 selon l'invention, améliore le score visuel de l'état de surface de la lésion dermique à l'origine de la vergeture blanche.

### Evaluation de l'efficacité et de la tolérance de l'application d'une composition cosmétique selon la formule 1 pour traiter les vergetures rosées

On applique le même protocole que celui de l'exemple 1 si ce n'est que les patients traités sont des patients présentant des vergetures rosées.

L'évaluation est conduite sur 29 individus constitués d'hommes (1) et de femmes (28).

Le tableau 2 ci-dessous montre le pourcentage moyen d'amélioration observée de la surface de chacune des vergetures rosées évaluées, en fonction du nombre de jours de traitement et pour chacune des compositions testées.

**Tableau 2**

| | % d'amélioration de la surface des vergetures rosées | |
|---|---|---|
| Nb de jours de traitement | Avec la composition de l'invention de formule 1 | Avec al composition « placebo » |
| 30 jours | +2,34% | -1.28% |
| 60 jours | +1.72% | +0.37% |
| 90 jours | +2.65% | +1.33% |

Le tableau 2 montre que l'application de la composition de formule 1 de l'invention ou du placebo n'améliore pas la lésion des vergetures rosées.

L'évaluation visuelle de la lésion sur la zone traitée par la composition de l'invention, réalisée sur chaque individu, confirme ces résultats.

Après 90 jours de traitement, aucune amélioration n'est observée.

Ainsi, la composition de l'invention a pour effet d'améliorer l'état et l'aspect visuel des vergetures blanches existantes. La composition de l'invention permet également de prévenir l'apparition de nouvelles vergetures blanches, mais aussi de ralentir le développement de celles existantes, en diminuant la surface globale qu'elles occupent sur la peau.

En plus de son efficacité démontrée même à faible concentration, le palmitoyl-tripeptide-5, en tant que principe actif dermatologique d'une composition pour les vergetures blanches, est facile à formuler.

L'usage du palmitoyl-tripeptide-5, dans une composition pour prévenir et/ou traiter spécifiquement les vergetures blanches, est également facile à même en œuvre et peu couteux.

Ainsi, la composition de l'invention, pour prévenir et/ou traiter les seules vergetures blanches, comprenant en tant que principe actif dermatologique, du palmitoyl-tripeptide-5 consiste en une solution efficace, peu couteuse, utilisable par tous type de sujet, facile à mettre en œuvre pour prévenir et/ou traiter les vergetures blanches de manière ciblée.

## Revendications

1. Composition comprenant du palmitoyl tripeptide -5 comme seul et unique principe actif dermatologique, lequel représente entre 0.01% et 0.1% en masse par rapport à la masse totale de la composition pour utilisation dans la prévention et/ou le traitement des vergetures blanches.

2. Composition, pour son utilisation, selon la revendication précédente, **caractérisée en ce que** ledit palmitoyl tripeptide -5 représente 0,025% en masse par rapport à la masse totale de la composition.

3. Composition selon l'une quelconque des revendications précédentes, pour son utilisation dans la prévention et/ou le traitement des seules vergetures blanches.

4. Composition, pour son utilisation, selon l'une quelconque des revendications précédentes, **caractérisée en ce qu'**elle se présente sous une forme galénique adaptée pour un usage topique.

5. Composition, pour son utilisation, selon l'une quelconque des revendications précédentes, **caractérisée en ce qu'**elle se présente sous une forme adaptée pour une voie d'administration cutanée.

6. Composition, pour son utilisation, selon l'une quelconque des revendications précédentes, **caractérisée en ce qu'**elle se présente sous la forme de crème, de gel, d'huile, de lotion, de solution, de pommade, de mousse, d'émulsion.

7. Composition, pour son utilisation, selon l'une quelconque des revendications précédentes, **caractérisée en ce qu'**il s'agit d'une composition cosmétique.

8. Procédé cosmétique, pour la prévention et/ou le traitement des vergetures blanches, selon lequel on applique sur les vergetures blanches une composition selon l'une quelconque des revendications 1 à 7.
